# EUROPEAN PATENT APPLICATION

(11) **EP 1 548 440 A1**
(43) Date of publication of application: **29.06.2005**
(21) Application number: 03797639.6
(22) Date of filing: 17.09.2003
(51) Int. Cl.: G01N 33/543, G01N 33/553, G01N 33/53

(54) **PARTICLE COMPOSITE AND PROCESS FOR PRODUCING PARTICLE COMPOSITE**

(30) Priority: 17.09.2002 JP 2002270813
(71) Applicant: Universal Bio Research Co., Ltd., Matsudo-shi, Chiba 271-0064 (JP)
(72) Inventor: TAJIMA, Hideji, Universal Bio Research Co., Ltd., Matsudo-shi, Chiba 271-0064 (JP); STIMPSON, Donald, I., Haviland, KS 67059 (US)
(74) Representative: Popp, Eugen, Dr.
(86) International application number: PCT/JP2003/011854
(87) International publication number: WO 2004/027425

(57) **Abstract**

It is an object of the present invention to provide a particle complex that is dispersible in a liquid sample containing a target substance, and that comprises a first particle on which a reactive substance capable of reacting with a target substance is immobilized and a second particle labeled with a labeling substance, and wherein it is effectively prevented that the second particle will become a steric hindrance and decrease the efficiency of the reaction between the reactive substance and the target substance, and to achieve the object, the present invention provides a particle complex wherein a first particle on which a reactive substance capable of reacting with a target substance is immobilized and a second particle labeled with a labeling substance are immobilized on the surface of a solid support that is dispersible in a liquid sample containing the target substance.

## Description

### TECHNICAL FIELD

The present invention relates to a particle complex comprising a first particle on which a reactive substance capable of reacting with a target substance is immobilized and a second particle labeled with a labeling substance, and a method for producing the particle complex.

### BACKGROUND ART

A solid support on which a probe comprising a base sequence complementary to a target gene sequence is immobilized, is used in the process of analyzing expression, mutation, polymorphism, etc. , of the target gene. Analysis of the target gene is performed, for example, by bringing the solid support having the immobilized probe into contact with a liquid sample containing the target gene, and then detecting the presence or absence of hybridization between the probe and the target gene. In such a process, the reaction efficiency between the target gene and the probe can be increased by using particles as the solid support having the immobilized probe and dispersing the particles having the immobilized probe in the liquid sample containing the target gene.

High throughput analysis such as high throughput screening becomes possible if a plurality of particles having various different types of immobilized probes are used, and a plurality of reaction types between the target gene and the probes can be performed concurrently in a single liquid sample; in order to realize this kind of high throughput analysis, however, the type of probe immobilized on each particle must be identifiable.

For example, a composite particle wherein a fluorescently labeled nanoparticle is attached to the surface of microparticle on which a probe is immobilized has been disclosed (Published Japanese Translations of PCT International Publication No. 2002-501184, and when this composite particle is used, the type of probe immobilized on the microparticle can be identified based on the fluorescent label of the nanoparticle.

### DISCLOSURE OF THE INVENTION

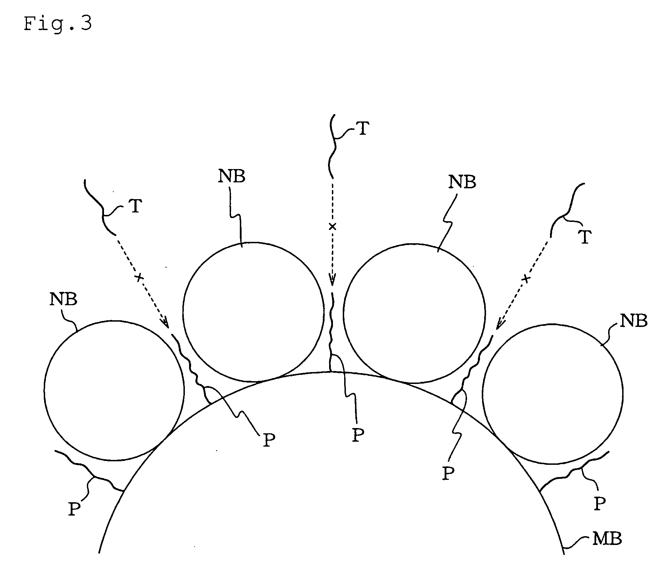

On the surface of the microparticle, however, it is extremely difficult to control the region whereto the probe is attached and the region whereto the fluorescently labeled nanoparticle is attached. Therefore, as shown in Figure 3, the probes P immobilized on the surface of the microparticle MB are covered by the nanoparticles NB that are attached to the surface of the microparticle MB, and there is a concern that the nanoparticles NB will become a steric hindrance and decrease the efficiency of the reaction between the probes P immobilized on the surface of the microparticle MB and the target genes T. In such a case, the significance of using a particle as a solid support for an immobilized probe is lost.

Thus, first of all, an object of the present invention is to provide a particle complex that is dispersible in a liquid sample containing a target substance, and that comprises a first particle on which a reactive substance capable of reacting with the target substance is immobilized and a second particle labeled with a labeling substance, and wherein it is effectively prevented that the second particle will become a steric hindrance and decrease the efficiency of the reaction between the reactive substance and the target substance.

A further object of the present invention is to provide a method for producing a particle complex, thus enabling efficient production of the aforementioned particle complex.

To accomplish the aforementioned objects, the present invention provides the following particle complex and method for producing the same.
(1) A particle complex, wherein a first particle on which a reactive substance capable of reacting with a target substance is immobilized and a second particle labeled with a labeling substance are immobilized on the surface of a solid support dispersible in a liquid sample containing the target substance.
(2) The particle complex according to (1) above, wherein plural first particles and plural second particles are immobilized on the surface of the solid support.
(3) The particle complex according to (2) above, wherein the ratio of the first particle size to the second particle size is 1 or greater.
(4) The particle complex according to (1) above, wherein a correlation is established between the type of the reactive substance and the labeling by the labeling substance.
(5) The particle complex according to (4) above, wherein plural second particles labeled with different types of labeling substances are immobilized on the surface of the solid support, and a correlation is established between the type of the reactive substance and the combination and/or the quantitative ratio of the different types of the labeling substances.
(6) The particle complex according to (1) above, wherein one or more of the first particle, the second particle and the solid support possess magnetism.
(7) The particle complex according to (6) above, wherein the solid support does not possess magnetism, and one or more of the first particle and the second particle possess magnetism.
(8) The particle complex according to (1) above, wherein the labeling substance is a fluorescent substance, the ratio of the first particle size to the second particle size is greater than 1, and the first particle possesses optical transparency.
(9) The particle complex according to (8) above, wherein the first particle does not possess magnetism, and one or more of the second particle and the solid support possess magnetism.
(10) The particle complex according to (9) above, wherein the solid support does not possess magnetism and the second particle possesses magnetism.
(11) The particle complex according to (1) above, wherein the solid support is a particle or a cut fragment of a member with an elongated shape.
(12) The particle complex according to (1) above, wherein the first particle and the second particle are immobilized on the surface of the solid support by adhesive force of a polymer that can exhibit the adhesive force by going through a dry state.
(13) The particle complex according to (1) above, wherein the reactive substance is a biological substance.
(14) The particle complex according to (13) above, wherein the biological substance is a nucleic acid or a protein.
(15) A method for producing a particle complex, comprising
   a step for immobilizing a first particle on which a reactive substance capable of reacting with a target substance is immobilized and a second particle labeled with a labeling substance on predetermined regions of the surface of a solid support, and
   a step for cutting the solid support into microfragments that contain part or all of the regions and that are dispersible in a liquid sample containing the target substance.
(16) The production method according to (15) above, wherein the solid support is a member with an elongated shape.
(17) The production method according to (16) above, wherein the first particle and the second particle are immobilized on the entire surface of the member with an elongated shape, and the member with an elongated shape is then cut into plural microfragments.
(18) The production method according to (15) above, wherein the first particle and the second particle are immobilized on the surface of the solid support by adhesive force of a polymer that can exhibit the adhesive force by going through a dry state.

### BRIEF DESCRIPTION OF THE DRAWINGS

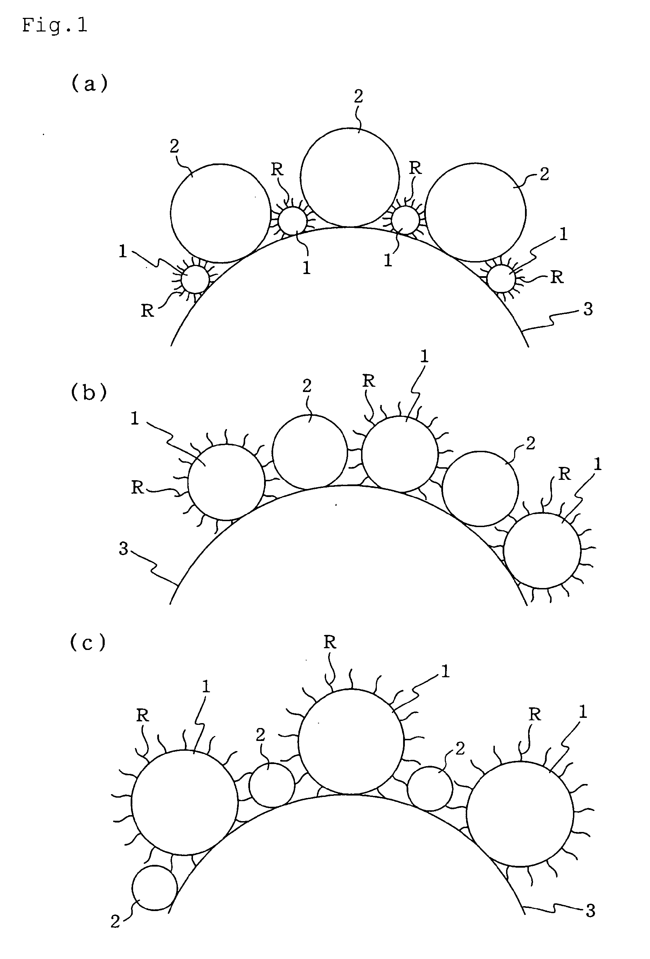

Figure 1 is a schematic drawing explaining that in the particle complex of the present invention, as the ratio of the first particle size to the second particle size (the first particle size/the second particle size) grows larger, it is increasingly difficult for the second particle to become a steric hindrance, thereby effectively preventing the decrease in efficiency of the reaction between the reactive substance and the target substance.

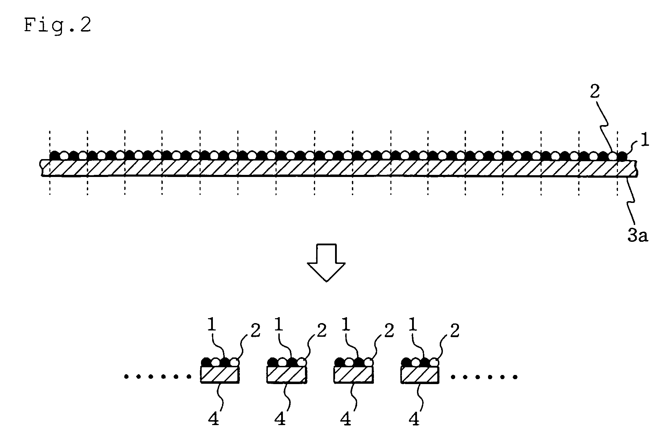

Figure 2 is an explanatory drawing of a case in which plural particle complexes are produced using materials with an elongated shape.
Figure 3 is a schematic drawing explaining that in a composite particle in which fluorescently labeled nanoparticles NB are attached to the surface of a microparticle MB on which probes P have been immobilized, the probes P immobilized on the surface of the microparticle MB are covered by the nanoparticles NB that are attached to the surface of the microparticle MB, and the nanoparticles NB become a steric hindrance and decrease the efficiency of the reaction between the probes P immobilized on the surface of the microparticle MB and the target genes T.

### BEST MODE FOR CARRYING OUT THE INVENTION

The particle complex and the method for producing the same of the present invention are described in detail below.

In the particle complex of the present invention, the first particle and the second particle are immobilized on the surface of a solid support that is dispersible in a liquid sample.

The term "particle" refers to a minute, three-dimensional structure wherein a reactive substance, labeling substance, etc., can be immobilized on the surface thereof, but is not restricted in the present invention with respect to shape, size, etc. The particle shape, for example, can be spherical with preferred particle diameter of approximately 0.1 µm to approximately 100 µm.

The particle material is one that is insoluble in the sample liquid and that can be selected as needed in response to the type of solvent used in the sample liquid, etc. Particle materials include, for example, polymers obtained by the polymerization of one or more vinyl monomers such as aromatic vinyl compounds, α,β-unsaturated carboxylic acid esters or amides, α,β-unsaturated nitriles, halogenated vinyls, conjugated dienes, lower fatty acid vinyl esters, etc., such as styrene, chlorstyrene, chloromethyl styrene, α-methyl styrene, divinyl benzene, sodium styrene sulfonate, (meth)acrylate, methyl (meth)acrylate, ethyl (meth)acrylate, n-butyl (meth)acrylate, 2-hydroxyethyl (meth)acrylate, polyoxyethylene (meth)acrylate, glycidyl (meth)acrylate, ethylene glycol di(meth)acrylate, tribromophenyl (meth)acrylate, tribromopropyl acrylate, (meth)acrylonitrile, (meth)acrolein, (meth)acrylamide, methylene-bis-(meth)acrylamide, butadiene, isoprene, vinyl acetate, vinyl pyridine, N-vinyl pyrrolidone, vinyl chloride, vinyl bromide, etc.; crosslinked polysaccharides such as agarose, dextran, cellulose, carboxymethyl cellulose, etc.; crosslinked proteins such as methylated albumin, gelatin, collagen, casein, etc.; inorganic materials such as glass, ceramic, etc.; metals such as iron, silicon, etc.; and composites thereof, etc. It is preferable that the particle material has non-swelling properties, but optionally it may have swelling properties. The surface of the particles may be porous or nonporous, but when the surface of the particles is porous, a larger amount of reactive substance, labeling substance, etc., can be immobilized on the surface thereof than when it is nonporous.

The term "solid support" refers to a three-dimensional structure wherein the first particle and the second particle can be immobilized upon the surface thereof.

The material of the solid support is one that is insoluble in the sample liquid and that can be selected as needed in response to the type of solvent used in the sample liquid, etc. Examples of the material of the solid support include, for example, plastic (for example, polyethylene, polypropylene, polyamide, polyvinylidene difluoride, etc.); metals (for example, iron, gold, silver, copper, aluminum, nickel, cobalt, silicon, etc.); glass; ceramic; and composites thereof, etc. It is preferable that the solid support material has non-swelling properties, but optionally it may have swelling properties. The surface of the solid support may be porous or nonporous, but when the surface of the solid support is porous, a larger number of particles can be immobilized on the surface thereof than when it is nonporous.

Examples of the solid support dispersible in a sample liquid include a particle or a cut fragment of a member with an elongated shape. Examples of a member with an elongated shape include a member that is filamentous, fibrous, rod-shaped, tape-shaped, etc. When the solid support is a particle, the preferred particle diameter is approximately 10 µm to approximately 1000 µm, and when the solid support is a cut fragment of a member with an elongated shape, the preferred fragment length is approximately 10 µm to approximately 2000 µm.

The term "surface of the solid support" refers to a surface capable of contact with the liquid sample, and naturally this term includes both the external surface (outer surface) of the solid support and internal surface (inner surface) of the solid support that can be permeated by the liquid sample (for example, the inner surfaces of pores in the solid support).

The number of the first particle or the second particle immobilized on the surface of the solid support is not restricted in the present invention, and may be one or more than one, but it is preferred that the number of the first particle is more than one and the number of the second particle is more than one. When plural first particles are immobilized on the solid support, the type of reactive substance may differ or may be identical among particles or groups of particles. In addition, when plural second particles are immobilized on the solid support, the type of label may differ or may be identical among particles or groups of particles depending on the labeling substance (for example, different combinations or quantitative ratios of labeling substances).

It is preferred that the size of the solid support is greater than the sizes of both the first particle and the second particle such that a plurality of both first particles and second particles can be immobilized on the surface of the solid support.

When plural first particles and plural second particles are immobilized on the surface of the solid support, it is possible that the second particles will cover the first particles and become a steric hindrance, decreasing the efficiency of the reaction between the reactive substance and the target substance thereby, but as shown in Fig. 1(a)-(c), as the ratio of the first particle size to the second particle size grows larger, it is increasingly difficult for the second particle to become a steric hindrance, and the decrease in efficiency of the reaction between the reactive substance and the target substance can be effectively prevented thereby. More specifically, rather than a case in which when the particle size of the first particles is smaller than that of the second particles (Fig. 1(a)), the decrease in the aforementioned reaction efficiency can be prevented more effectively when the particle size of the first particles is approximately the same as the particle size of the second particles (Fig. 1(b)), and the decrease in the aforementioned reaction efficiency can be prevented even more effectively when the particle size of the first particles is greater than that of the second particles (Fig. 1(c)). In Figure 1, 1 represents the first particles, 2 represents the second particles, 3 represents the solid support, and R represents the reactive substance.

Therefore, when plural first particles and plural second particles are immobilized on the surface of the solid support, from the standpoint of preventing a decrease in the efficiency of the reaction between the reactive substance and the target substance, it is preferable to set the particle sizes of the first particles and second particles such that the ratio of the first particle size to the second particle size will be large; thus, the ratio of the first particle size to the second particle size is preferably 1 or more, and more preferably 2 or more.

In the particle complex of the present invention, it is preferable for one or more of the first particle, second particle and solid support to possess magnetism. For example, it is possible to make the particles or solid support magnetic by including magnetic substances such as iron hydroxide, iron oxide hydrate, γ-Fe₂O₃, Fe₃O₄, etc., in the particles or the solid support. When one or more of the first particle, second particle or solid support is magnetic, it is possible to control the behavior of the particle complex of the present invention easily by the use of magnetic force; therefore, automation of the analysis of the target substance by the use of the particle complex of the present invention can easily be realized.

When the particles or the solid support contain a magnetic substance, it is possible that chemical modification of the surface of the particles or solid support will be incomplete, thereby decreasing the immobilization efficiency of the reactive substance to the first particle, the immobilization efficiency of the labeling substance to the second particle, or the immobilization efficiency of the first particle and second particle to the solid support. In the case of the first and second particles, even if the immobilization efficiency of the reactive substance or target substance in each respective particle is decreased, it is possible to offset the decrease in immobilization efficiency in each respective particle by increasing the number of particles immobilized on the solid support, but in the case of the solid support it is impossible to offset the decrease in immobilization efficiency of the first and second particles. Therefore, it is preferable to prevent a decrease in immobilization efficiency of the first and second particles on the solid support by not including a magnetic substance in the solid support. More specifically, from the standpoint of preventing a decrease in the immobilization efficiency of the first and second particles on the solid support, it is preferable that the solid support dose not possess magnetism, and that one or more of the first particle or second particle possess magnetism.

In the particle complex of the present invention, a reactive substance capable of reacting with a target substance is immobilized on the surface of the first particle.

The term "surface of the first particle" refers to a surface capable of contact with the liquid sample, and naturally this term includes both the external surface (outer surface) of the particle and the internal surface (inner surface) of the particle that can be permeated by the liquid sample (for example, the inner surfaces of pores in the particle).

The term "target substance" refers to a substance that can be subjected to detection, isolation, analysis, etc.; thus, a substance in which the structure and function, etc., are publicly known or unknown can be selected as needed in accordance with the purpose of the test, inspection, analysis, etc., to be performed using the particle complex of the present invention. The present invention does not restrict the type of target substance, and concrete examples include biological substances such as nucleic acids, proteins, antigens, antibodies, enzymes, sugar chains, etc. The term "nucleic acid" includes both DNA and RNA, and also analogues and derivatives thereof (for example, a peptide nucleic acid (PNA), phosphothioate DNA, etc.) The present invention does not restrict the base length of the nucleic acid, and it may be either an oligonucleotide or a polynucleotide. The nucleic acid may be either single stranded, double stranded, or a mixture thereof.

The term "reactive substance" refers to a substance capable of reacting with the target substance, and a substance which has reactivity with the target substance or a substance which may have reactivity with the target substance can be selected as needed in accordance with the purpose of the test, inspection, analysis, etc., to be performed using the particle complex of the present invention. The reactive substance can be a substance in which the structure and function, etc., are publicly known or unknown. The present invention does not restrict the type of reactive substance, and concrete examples include biological substances such as nucleic acids, proteins, antigens, antibodies, enzymes, sugar chains, etc. The reactivity that the reactive substance has (or may have) with the target substance may be any kind of reactivity, and this includes, for example, the property of attaching with the target substance by a coupling scheme such as a covalent bond, ionic bond, van der Waals force, hydrogen bond, coordination bond, chemical adsorption, physical adsorption, etc.

Concrete examples of combinations of target substance and reactive substance include nucleic acid/complementary nucleic acid, receptor protein/ligand, enzyme/substrate, antibody/antigen, etc.

The present invention dose not restrict the number of reactive substances immobilized on the surface of the first particle, but it is preferred that a plurality of reactive substances be immobilized on the surface of the first particle, i.e., it is preferred that the reactive substances on the surface of the first particle be integrated. When a plurality of reactive substances are immobilized on the surface of the first particle, the type of reactive substance may be the same or different, but normally it is the same.

In the particle complex of the present invention, the second particle is labeled with a labeling substance.

The labeling substance may be attached to the surface of the second particle, or it may be embedded within the second particle.

The present invention does not restrict the type of labeling substance, and concrete examples include fluorescent substances such as fluorescent dyes (for example, Marine Blue, Cascade Blue, Cascade Yellow, Fluorescein, Rhodamine, Phycoerythrin, CyChrome, PerCP, Texas Red, Allophycocyanin, PharaRed, etc., as well as Cy dyes such as Cy2, Cy3, Cy3.5, Cy5, Cy7, etc., Alexa dyes such as Alexa-488, Alexa-532, Alexa-546, Alexa-633, Alexa-680, etc., and BODIPY dyes such as BODIPY FL, BODIPY TR, etc.), radioactive substances such as radioactive isotopes (for example, ³H, ¹⁴C, ³²P, ³³P, ³⁵S, and ¹²⁵I), etc. When a fluorescent dye is used as the labeling substance, a variety of labels become possible by combining different types and quantitative ratios of the fluorescent dyes. Detection of the fluorescence emitted by the fluorescent dye can be performed using flow cytometry, for example.

Labeling with a fluorescent dye can be performed, for example, by reacting a fluorescent dye having an active ester with a particle wherein an amino group has been previously introduced onto the surface thereof, or by reacting a fluorescent dye having a functional group capable of a bonding reaction with a carboxyl group (for example, an amino group) or a fluorescent dye having a functional group capable of a bonding reaction with an amino group (for example, a carboxyl group) with a particle wherein a carboxyl group or an amino group has been previously introduced onto the surface thereof, in the presence of a carbodiimide such as 1-ethyl-3-(3-dimethylamino-propyl)-3-ethylcarbodiimide hydrochloride (EDC), etc. Moreover, labeling of a particle with a fluorescent dye can be performed when synthesizing the particles in a polymerization reaction by adding a fluorescent dye to the reaction liquid, or immediately after the completion of a polymerization reaction by radical polymerization, by adding a fluorescent dye having reactivity with the radical while the radical is still present.

In the particle complex of the present invention, the type of reactive substance can be identified based on the label of the labeling substance by establishing a correlation between the type of reactive substance and the label of the labeling substance. With a plurality of particle complexes in which different types of reactive substances have been immobilized thereon, if it is possible to identify the type of reactive substance immobilized on each particle complex based on the label of the labeling substance thereon, it will be possible to analyze a plurality of types of reactions between the reactive substance and the target substance concurrently, thereby enabling high throughput analysis such as high throughput screening, etc. For example, by immobilizing a plurality of second particles labeled with different types of labeling substances on the surface of a solid support and establishing a correlation between the type of reactive substance and the combination and/or the quantitative ratio of the different types of labeling substances, the type of reactive substance immobilized on each particle complex can be identified based on the label of the labeling substance. In addition, by altering the combination and/or the quantitative ratio of the labeling substances, a variety of labels can be obtained even when using a small number of labeling substances.

In the particle complex of the present invention, the precision of detection of the fluorescent light emitted by the fluorescent dye may be decreased when the labeling substance that labels the second particle is a fluorescent dye, the ratio of the first particle size to the second particle size is greater than 1, and the first particle does not transmit light. More specifically, when the ratio of the first particle size to the second particle size is greater than 1, because the second particles are covered by the first particles (see Fig. 1(c)), the fluorescent light emitted by the fluorescent dye contained in the second particles may be blocked by first particle that do not transmit light. Therefore, from the standpoint of preventing a decrease in the precision of the detection of the fluorescent light, it is preferable for the first particle to possess optical transparency when the labeling substance that labels the second particle is a fluorescent dye, and the ratio of the first particle size to the second particle size is greater than 1.

In the particle complex of the present invention, the optical transparency of the first particle may be decreased by the magnetic substance contained therein when the first particle possesses magnetism. Therefore, from the standpoint of preventing a decrease in the precision of the detection of the fluorescent light, when the labeling substance that labels the second particle is a fluorescent dye, and the ratio of the first particle size to the second particle size is greater than 1, it is preferable for the first particle not to possess magnetism. Thus, from the standpoint of increasing the workability of the particle complex of the present invention, it is preferable for one or more of the second particle and the solid support to possess magnetism, and furthermore, from the standpoint of preventing a decrease in immobilization efficiency of the first particle and the second particle on the solid support, it is preferable for the second particle to possess magnetism and for the solid support not to possess magnetism.

In the particle complex of the present invention, immobilization of the first particle and the second particle on the solid support and immobilization of the reactive substance on the first particle can be performed by various coupling schemes. Concrete examples of coupling schemes include the specific interactions of streptoavidin-biotin and avidin-biotin, hydrophobic interactions, magnetic interactions, polar interactions, formation of a covalent bond (for example, amide bond, disulfide bond, thioether bond, etc.), and crosslinking by a crosslinking agent, etc. Suitable chemical modification of the surface of the solid carrier, surfaces of the particles, or the reactive substance can be performed using publicly known techniques to enable immobilization by these coupling schemes. Immobilization of the reactive substance on the first particle is performed prior to immobilization of the first particle on the solid support.

Immobilization of the first particle and immobilization of the second particle on the solid support, and immobilization of the reactive substance on the first particle can be performed using specific interactions other than streptoavidin-biotin and avidin-biotin such as maltose binding protein/maltose, polyhistidine peptide/metal ion such as nickel and cobalt, glutathione-S-transferase/glutathione, calmodulin/calmodulin binding peptide, ATP binding protein/ATP, nucleic acid/complementary nucleic acid, receptor protein/ligand, enzyme/substrate, antibody/antigen, IgG/protein A, etc.

It is preferable that the coupling scheme between the first and second particles and the solid support, and the coupling scheme between the reactive substance and the first particle be a coupling scheme such that the coupling partners (solid support and particle, or particle and reactive substance) do not easily separate. This kind of coupling scheme includes, for example, avidin-biotin and streptoavidin-biotin interactions, formation of covalent bonds, crosslinking by a crosslinking agent, adhesion by a polymer that can exhibit adhesive force by going through a dry state, adhesion by a polymer that can exhibit adhesive force through exposure to UV light, etc.

When the avidin-biotin or streptoavidin-biotin interaction is used, for example, a particle coated with avidin or streptoavidin can be bound to a solid support coated with biotin. In addition, a reactive substance wherein biotin has been introduced (for example, a biotinized nucleic acid obtained by performing PCR using a primer that has been biotinized on the 5' terminus) can be bound to a particle coated with avidin or streptoavidin. It is possible to reverse the sites of avidin or streptoavidin and biotin, for example, and bind a particle coated with biotin to a solid support coated with avidin or streptoavidin. Binding of the particle and the reactive substance can be performed in the same manner.

When the formation of a covalent bond is used, it is possible to form a covalent bond using a functional group that is present on the surface of the solid support, on the surface of the particle, or in the reactive substance. Concrete examples of functional groups that can form covalent bonds include a carboxyl group, amino group, hydroxyl group, etc. For example, when a carboxyl group is present on the surface of the solid support, it is possible to form an amide bond between the solid support and a particle by activating the carboxyl group with a carbodiimide such as 1-ethyl-3-(3-dimethyl amino-propyl)-3-ethylcarbodiimide hydrochloride (EDC), etc., and then reacting it with an amino group present on the surface of the particle. In addition, when an amino group is present on the surface of the solid support, it is possible to form an amide bond between the solid support and a particle by replacing the amino group with a carboxyl group using a cyclic acid anhydride such as succinic anhydride, and then reacting it with the amino group present on the surface of the particle. Bonding of the particle and the reactive substance can be performed in the same manner. Moreover, when the reactive substance is a nucleic acid, it is preferable that the nucleic acid be coupled with the particle via a linker sequence introduced to either the 5' terminus or 3' terminus of the nucleic acid so that the reactivity of the nucleic acid (hybridization capability with a complementary nucleic acid) is not lost.

When crosslinking by a crosslinking agent is used, various crosslinking agents capable of reacting with functional groups on the substances to be crosslinked can be used. Concrete examples of crosslinking agents include polyfunctional reagents such as bifunctional reagents, trifunctional reagents, etc. Concrete examples of these kinds of polyfunctional reagents include N-succinimidyl (4-iodoacetyl)aminobenzoate (SIAB), dimaleimide, dithio-bis-nitrobenzoic acid (DTNB), N-succinimidyl-S-acetylthioacetate (SATA), N-succinimidyl-3-(2-pyridyldithio) propionate (SPDP), succinimidyl 4-(N-maleimidomethyl) cyclohexane-1-carboxylate (SMCC), 6-hydrazinonicotinamide (HYNIC), etc.

When adhesive of a polymer that can exhibit adhesive force by going through a dry state is used, it is possible to immobilize a particle to the surface of the solid support easily by interspersing the wet polymer between the solid support and the particle, and then drying the polymer. Examples of polymers that can exhibit adhesive force by going through a dry state include proteins, polyvinyl alcohols, etc. Naturally, the adhesive force that these polymers can exhibit in a dry state is maintained as long as the dry state continues, and it is also maintained when a liquid infiltrates the surface of the solid support whereupon the particle is immobilized. Either a particle, the solid support, or both may contain a polymer that can exhibit adhesive force by going through a dry state. Moreover, it is possible that neither the particle nor the solid support will contain the polymer, and the polymer is added when the particle is immobilized on the solid support.

In the particle complex of the present invention, because the first particle on which a reactive substance is immobilized and the second particle labeled with a labeling substance are immobilized on the surface of the solid support as separate particles, the first particle and the second particle both constitute the surface of the particle complex of the present invention. In other words, the reactive substance immobilized on the surface of the first particle is in an exposed state. As a result, in the particle complex of the present invention this effectively can prevent the second particle from becoming a steric hindrance and decreasing the efficiency of the reaction between the reactive substance and the target substance. As noted above, this effectiveness in the particle complex of the present invention increases as the ratio of the first particle size to the second particle size grows larger Because the first particle, second particle, and solid support that constitute the particle complex of the present invention are all dispersible in the liquid sample, the particle complex of the present invention is entirely dispersible in the liquid sample. Therefore, the reactive substance and the target substance can be reacted efficiently by dispersing the particle complex of the present invention in a liquid sample containing the target substance.

The particle complex of the present invention can be produced by immobilizing a first particle on which a reactive substance capable of reacting with a target substance is immobilized and a second particle labeled with a labeling substance on predetermined regions of the surface of a solid support, and cutting the solid support into microfragments that contain part or all of the regions and that are dispersible in a liquid sample containing the target substance.

More specifically, as shown in Figure 2, plural particle complexes can be produced by using a member with an elongated shape such as filamentous shape, fibrous shape, rod-shape, tape-shape, etc., as the solid support, and after the first particle and the second particle have been immobilized over the entire surface of the elongated material, cutting the elongated material into plural microfragments. In Figure 2, 1 represents first particles, 2 represents second particles, 3a represents the elongated material, and 4 represents the microfragments.

When immobilizing the first particles and the second particles over the entire surface of the elongated material, after the elongated material is immersed in a liquid containing the first particles and the second particles, specific immobilization processes may be performed either within the aforementioned liquid or after the elongated material is removed from the liquid.

When the first particles and the second particles are immobilized on the surface of the solid support using adhesive force of a polymer that can exhibit the adhesive force by going through a dry state, a process step in which the wet polymer is dried becomes necessary, but because handling of the particulate solid support outside of the liquid phase is very difficult, conducting the polymer drying process step becomes extremely difficult. However, because the solid support is easy to handle outside of the liquid phase prior to the formation of microfragments, the polymer drying process step can be easily performed at that time.

### INDUSTRIAL APPLICABILITY

In accordance with the present invention, a particle complex is provided that is dispersible in a liquid sample containing a target substance, and that comprises a first particle on which a reactive substance capable of reacting with a target substance is immobilized and a second particle labeled with a labeling substance, and wherein it is effectively prevented that the second particle will become a steric hindrance and decrease the efficiency of the reaction between the reactive substance and the target substance.

In addition, in accordance with the present invention, a method for producing a particle complex is provided, thereby enabling the aforementioned particle complex to be produced efficiently.

## Claims

1. A particle complex, wherein a first particle on which a reactive substance capable of reacting with a target substance is immobilized and a second particle labeled with a labeling substance are immobilized on the surface of a solid support dispersible in a liquid sample containing the target substance.

2. The particle complex according to claim 1, wherein plural first particles and plural second particles are immobilized on the surface of the solid support.

3. The particle complex according to claim 2, wherein the ratio of the first particle size to the second particle size is 1 or greater.

4. The particle complex according to claim 1, wherein a correlation is established between the type of the reactive substance and the labeling by the labeling substance.

5. The particle complex according to claim 4, wherein plural second particles labeled with different types of labeling substances are immobilized on the surface of the solid support, and a correlation is established between the type of the reactive substance and the combination and/or the quantitative ratio of the different types of the labeling substances.

6. The particle complex according to claim 1, wherein one or more of the first particle, the second particle and the solid support possess magnetism.

7. The particle complex according to claim 6, wherein the solid support does not possess magnetism, and one or more of the first particle and the second particle possess magnetism.

8. The particle complex according to claim 1, wherein the labeling substance is a fluorescent substance, the ratio of the first particle size to the second particle size is greater than 1, and the first particle possesses optical transparency.

9. The particle complex according to claim 8, wherein the first particle does not possess magnetism, and one or more of the second particle and the solid support possess magnetism.

10. The particle complex according to claim 9, wherein the solid support does not possess magnetism and the second particle possesses magnetism.

11. The particle complex according to claim 1, wherein the solid support is a particle or a cut fragment of a member with an elongated shape.

12. The particle complex according to claim 1, wherein the first particle and the second particle are immobilized on the surface of the solid support by adhesive force of a polymer that can exhibit the adhesive force by going through a dry state.

13. The particle complex according to claim 1, wherein the reactive substance is a biological substance.

14. The particle complex according to claim 13, wherein the biological substance is a nucleic acid or a protein.

15. A method for producing a particle complex, comprising
a step for immobilizing a first particle on which a reactive substance capable of reacting with a target substance is immobilized and a second particle labeled with a labeling substance on predetermined regions of the surface of a solid support, and
a step for cutting the solid support into microfragments that contain part or all of the regions and that are dispersible in a liquid sample containing the target substance.

16. The production method according to claim 15, wherein the solid support is a member with an elongated shape.

17. The production method according to claim 16, wherein the first particle and the second particle are immobilized on the entire surface of the member with an elongated shape, and the member with an elongated shape is then cut into plural microfragments.

18. The production method according to claim 15, wherein the first particle and the second particle are immobilized on the surface of the solid support by adhesive force of a polymer that can exhibit the adhesive force by going through a dry state.
